# EUROPEAN PATENT SPECIFICATION

(11) **EP 0 512 002 B1**
(45) Date of publication and mention of the grant of the patent: **21.08.1996**
(21) Application number: 91902866.2
(22) Date of filing: 24.01.1991
(51) Int. Cl.: A61K 47/48

(54) **CELL-SPECIFIC ENZYME-CONJUGATES FOR THE RELEASE OF TOXIC CYANIDE FROM CYANOGENIC PRODRUGS AT TARGET CELLS**
ZELL-SPEZIFISCHE ENZYMKONJUGATE, DIE TOXISCHES CYANIDE AUS CYANOGENEN VORLÄUFERDROGEN AN ZIELZELLEN FREISETZEN
CONJUGUES D'ENZYNES SPECIFIQUE POUR DES CELLULES, QUI LIBERENT DE LA CYANURE TOXIQUE DE PRODRUGS CYANOGENES DANS DES CELLULES CIBLES

(30) Priority: 24.01.1990 GB 9001641
(43) Date of publication of application: 11.11.1992
(73) Proprietor: IMPERIAL CANCER RESEARCH TECHNOLOGY LIMITED, London WC2A 3NL (GB)
(72) Inventor: EPENETOS, Agamemnon Antoniou, Imperial Cancer Res., Hospital, Du Cane Road, London W12 0HS (GB); ROWLINSON-BUSZA, Gail, Imperial Cancer Res. Fund, Du Cane Road, London W12 0HS (GB)
(74) Representative: Bassett, Richard Simon
(86) International application number: GB9100104
(87) International publication number: WO9111201

(56) References cited:
- EP-A- 0 302 473
- EP-A- 8 807 378
- WO-A-87/03205
- Plant Physiology, volume 94, 1990, J.E. Poulton: "Cyanogenesis in plants", pages 401-405
- STN File Server File Medline, AN 77040351
- Parker: "Immunologically directed cell destruction" in IMMUNOLOGIC INTERVENTION, 1971, pp. 196-206
- Parker: PHARMACOLOGICAL REVIEWS, vol. 25, 1973, pp. 325-342
- Parket et al.: "The immunologic therapy of cancer" in PHARMACOLOGY AND THE FUTURE OF MAN. Proc. 5th International Congress of Pharmacology, San Francisco, 1972, vol. 5, pp. 411-419 (Karger, Basel 1973)
- Lyle & Parker: FEDERATION PROCEEDINGS, vol. 33, 1974, pp. 1889-1893
- Philpott et al.: CANCER RESEARCH, vol. 39, 1979, pp. 2084-2089
- Moertel et al.: NEW ENGLAND JOURNAL OF MEDICINE, vol.306, no. 4, 1984, pp. 201-206

## Description

The present invention relates to potentially cytotoxic combinations of compounds which may be targeted to selected cells.

Bagshawe and his co-workers have disclosed (Bagshawe, *Br. J. Cancer* (1987) **56**, 531; Bagshawe *et al*, *Br. J. Cancer* (1988) **58**, 700; WO 88/07378) conjugated compounds comprising an antibody or part thereof and an enzyme, the antibody being specific to tumour cell antigens and the enzyme acting to convert an innocuous pro-drug into a cytotoxic compound. The pro-drug is administered with, or following, the conjugate and tumour cells are thereby killed. The cytotoxic compounds were alkylating agents, eg a benzoic acid mustard released from para-N-bis(2-chloroethyl)aminobenzoyl glutamic acid by the action of *Pseudomonas sp*. CPG2 enzyme. We have now devised improved systems using different pro-drugs.

EP 0 302 473; Parker (1971) "Immunologically directed cell destruction" in *Immunologic Intervention*, pp 196-206, Uhr, J.W. & Landy, M. (eds), Academic Press, NY, Parker (1973) *Pharmacol*. *Rev*. **25**, 325-342; Parker *et al* (1973) "The immunologic therapy of cancer" in *Pharmacology and the future of man*, pp 411-419, Proc. 5th International Congress of Pharmacology, San Francisco, Vol. 5, (Ed. R.A. Maxwell & G.H. Ackeson), S. Karger, NY; Lyle & Parker (1974) *Fed*. *Proc*. **33**, 1889-1893; and Philpott *et al* (1979) *Cancer Res*. **39**, 2084-2089 all disclose the general concept of antibody-enzyme conjugates in combination with pro-drugs for the delivery of cytotoxic agents to tumour cells but none disclose the use of a cyanogenic pro-drug in combination with an enzyme capable of generating cyanide from said cyanogenic pro-drug.

Moertel *et al* (1982) *New Eng. J.* Med. **306**, 201-206 discloses that amygdalin is a toxic drug that is not effective as a cancer treatment.

One aspect of the present invention provides a compound comprising a target cell-specific portion and an enzymatically active portion, the enzymatically active portion being capable of generating cyanide from a cyanogenic pro-drug. However, the compound is claimed *per se* only when the said enzymatically active portion is not a carbohydrate-cleaving enzyme capable of converting a glycosylated pro-drug into a free drug.

The liberated cyanide is toxic to cells in the vicinity (and, because it diffuses rapidly, may kill target cells which do not exhibit whatever is recognised by the compound but which are close to target cells which do exhibit what is recognised) but is quickly metabolised by endogenous enzymes, for example the rhodanase found in liver and kidney cells.

The entity which is recognised may be any suitable entity which is expressed by tumour cells, virally-infected cells, pathogenic microorganisms, cells introduced as part of gene therapy or even normal cells of the body which, for whatever reason, one wishes to destroy, but which entity is not expressed, or at least not with such frequency, in cells which one does not wish to destroy. The entity which is recognised will often be an antigen. Examples of antigens include those listed in Table 1 below. Monoclonal antibodies which will bind specifically to many of these antigens are already known (for example those given in the Table) but in any case, with today's techniques in relation to monoclonal antibody technology, antibodies can be prepared to most antigens. The antigen-specific portion may be an entire antibody (usually, for convenience and specificity, a monoclonal antibody), a part or parts thereof (for example an F_{ab} fragment or F(ab')₂) or a synthetic antibody or part thereof. A conjugate comprising only part of an antibody may be advantageous by virtue of being cleared from the blood more quickly and may be less likely to undergo non-specific binding due to the F_{C} part. Suitable monoclonal antibodies to selected antigens may be prepared by known techniques, for example those disclosed in "Monoclonal Antibodies: A manual of techniques", H Zola (CRC Press, 1988) and in "Monoclonal Hybridoma Antibodies: Techniques and Applications", J G R Hurrell (CRC Press, 1982). Bispecific antibodies may be prepared by cell fusion, by reassociation of monovalent fragments or by chemical cross-linking of whole antibodies, with the part of the resulting bispecific antibody being directed to the cell-specific antigen and the other to the enzyme. The bispecific antibody can be administered bound to the enzyme or it can be administered first, followed by the enzyme. Methods for preparing bispecific antibodies are disclosed in Corvalan *et al* (1987) *Cancer Immunol*. *Immunother*. **24**, 127-132 and 133-137 and 138-143. Bispecific antibodies, chimaeric antibodies and single chain antibodies are discussed generally by Williams in *Tibtech*, February 1988, Vol. 6 36-42, Neuberger *et al* (8th *International Biotechnology Symposium*, 1988, Part 2, 792-799) and Tan and Morrison (*Adv. Drug Delivery Reviews* 2, (1988), 129-142).

However, the bispecific antibody is claimed per se only when the said enzymatically active portion is not carbohydrate - cleaning enzyme capable of converting a glycosylated pro-drug into a free drug.

Suitably prepared non-human antibodies can be "humanized" in known ways, for example by inserting the CDR regions of mouse antibodies into the framework of human antibodies.

IgG class antibodies are preferred.

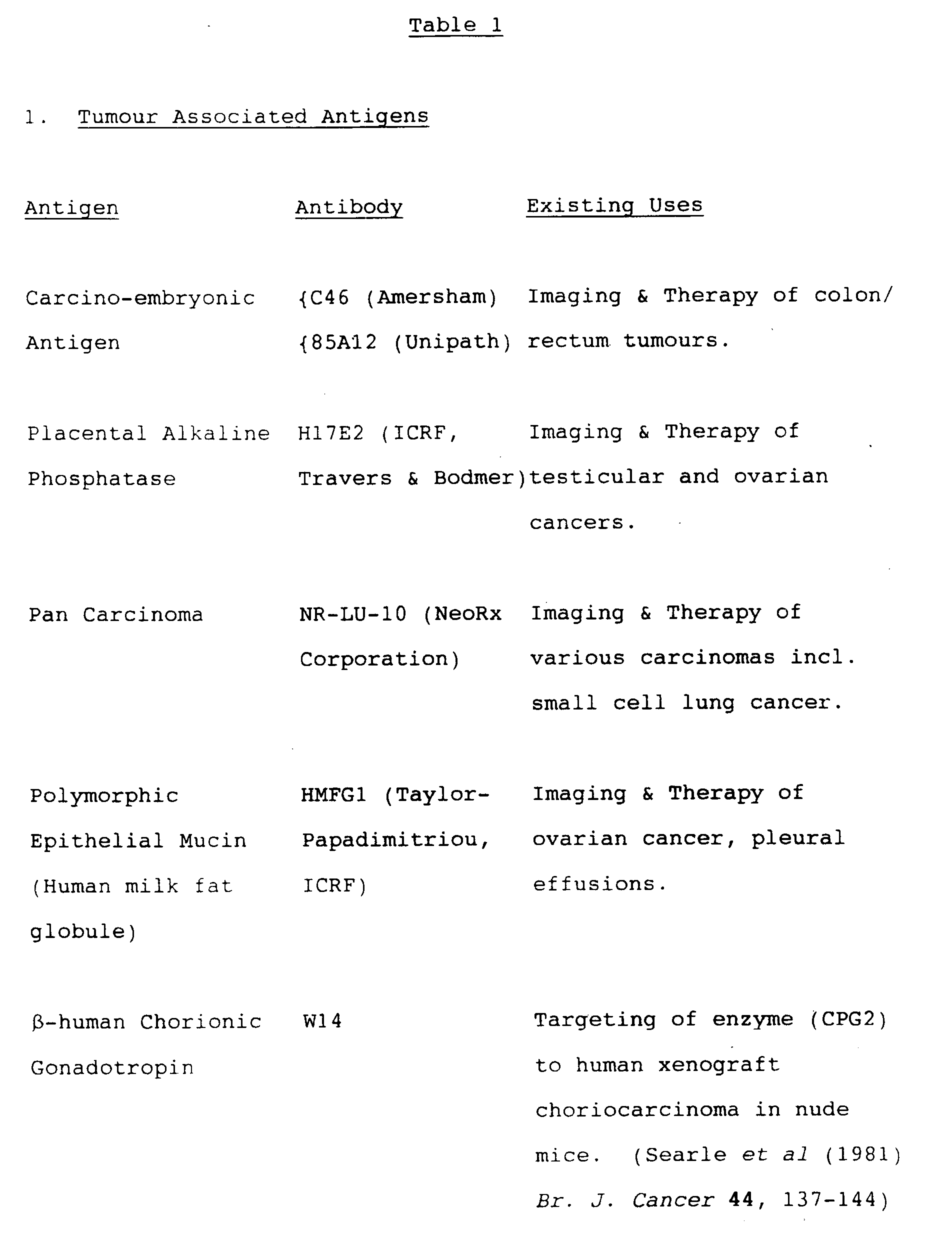

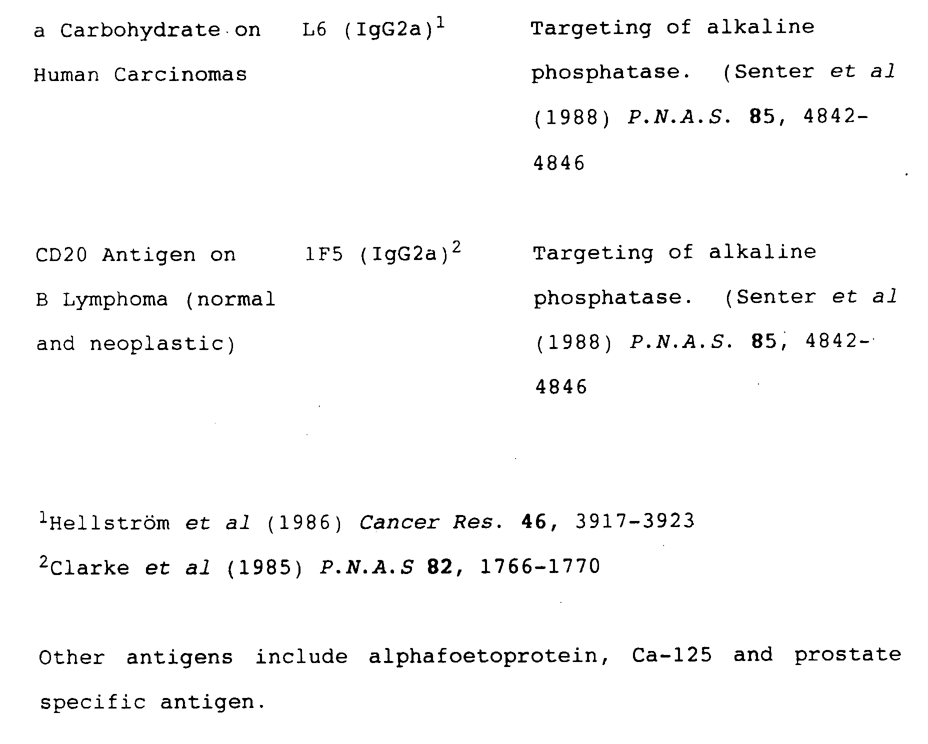

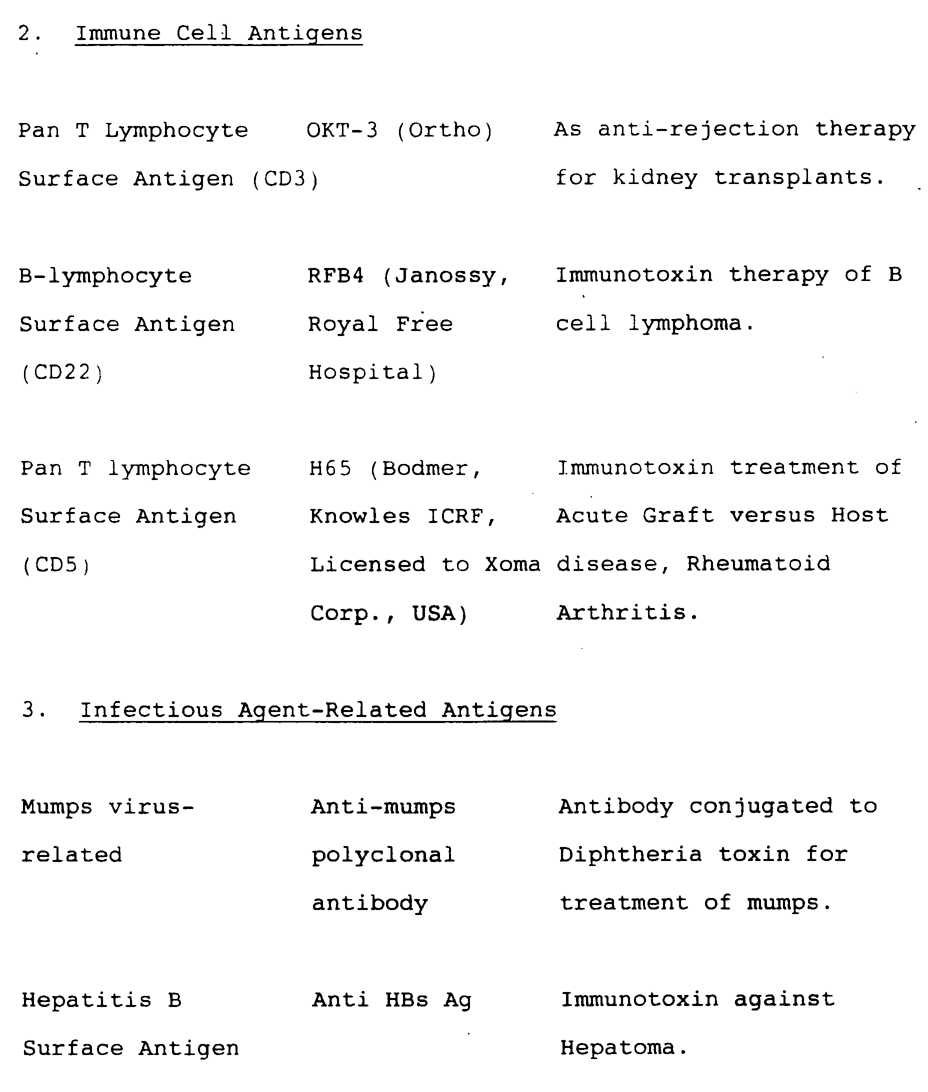

Alternatively, the entity which is recognised may or may not be antigenic but can be recognised and selectively bound to in some other way. For example, it may be a characteristic cell surface receptor such as the receptor for melanocyte-stimulating hormone (MSH) which is expressed in high numbers in melanoma cells. The cell-specific portion may then be a compound or part thereof which specifically binds to the entity in a non-immune sense, for example as a substrate or analogue thereof for a cell-surface enzyme or as a messenger. In the case of melanoma cells, the cell-specific portion may be MSH itself or a part thereof which binds to the MSH receptor. Such MSH peptides are disclosed in, for example, Al-Obeidi *et al* (1980) *J. Med. Chem.* **32**, 174. The specificity may be indirect: a first cell-specific antibody may be administered, followed by a compound of the invention directed against the first antibody. Preferably, the entity which is recognised is not secreted to any relevant extent into body fluids, since otherwise the requisite specificity may not be achieved.

The pro-drug may be any compound which is relatively non-toxic but which may be acted upon to yield cyanide. It is preferred that the non-cyanide product of the reaction is either non-toxic or, like the cyanide, is quickly metabolised to a non-toxic form on spreading from the site of action. Suitable compounds include amygdalin (D-mandelonitrile-β-D-glucosido-6-β-D-glycoside; [(6-O-β-D-glucopyranosyl-β-D-glucopyranosyl)oxy]benzene acetonitrile; "Laetrile"), or the DL racemic mixture, and dhurrin (hydroxyamygdalin), which are disaccharides, and prunasin and linamarin, which are both monosaccharides. Further cyanogenic compounds are being discovered all the time, often in plants. Amygdalin is preferred as it has been used in cancer therapy already (although without significant success) and is relatively non-toxic, at least when administered parenterally and non-concurrently with foods such as raw almonds which are rich in β-glucosidase.

The enzymatically active portion of the compound of the invention will be chosen by reference to the pro-drug. As in the case of the naturally-occurring pro-drugs themselves, more enzymes are being found all the time. Current examples include hydroxynitrile lyases from *Prunus amygdalus*, *P*. *laurocerasus*, *P*. *serotina*, *P*. *lyonii*, *Sorghum bicolor*, *S*. *vulgare*, *Linum usitatissimum*, *Manihot esculenta*, *Hevea brasiliensis* and *Davallia trichomanoides*. At least some of these are β-glycosidases, more particularly β-glucosidases, E.C. 3.2.1.21. These enzymes and their substrates are reviewed generally in the chapter entitled "Enzymology of plant cyanogenesis" by Poulton in "Cyanide Compounds in Biology" (Ciba Symposium No.140) Ed. Evered & Harnett, Wiley 1988. It is likely that the enzymatically active portion of the compound will be enzymatically active in isolation from the cell-specific portion but it is necessary only for it to be enzymatically active when (a) it is in combination with the cell-specific portion and (b) the compound is attached to or adjacent target cells.

The two portions of the compound of the invention are linked together by any of the conventional ways of cross-linking polypeptides, such as those generally described in O'Sullivan *et al Anal*. *Biochem*, (1979) **100** 100-108. For example, the antibody portion may be enriched with thiol groups and the enzyme portion reacted with a bifunctional agent capable of reacting with those thiol groups, for example the N-hydroxysuccinimide ester of iodoacetic acid (NHIA) or N-succinimidyl-3-(2-pyridyldithio)propionate (SPDP). Amide and thioether bonds, for example achieved with m-maleimidobenzoyl-N-hydroxysuccinimide ester, are generally more stable *in vivo* than disulphide bonds.

It may not be necessary for the whole enzyme to be present in the compound of the invention but, of course, the catalytic portion must be present. So-called "abzymes" may be used, where a monoclonal antibody is raised to a compound involved in the reaction one wishes to catalyse, usually the reactive intermediate state. The resulting antibody can then function as an enzyme for the reaction.

The antibody-enzyme conjugate may be purified by size exclusion or affinity chromatography, and tested for dual biological activities. The antibody immunoreactivity is measured using an enzyme-linked immunosorbent assay (ELISA) with immobilised antigen and in a live cell radio-immunoassay. An enzyme assay is used for β-glucosidase using a substrate which changes in absorbance when the glucose residues are hydrolysed, such as oNPG (o-nitrophenyl-β-D-glucopyranoside), liberating 2-nitrophenol which is measured spectrophotometrically at 405 nm.

Stability of the conjugate may be tested *in vitro* initially by incubating at 37°C in serum, followed by size exclusion FPLC analysis. Stability *in vivo* can be tested in the same way in mice by analysing the serum at various times after injection of the conjugate. In addition, it is possible to radiolabel the antibody with ¹²⁵I, and the enzyme with ¹³¹I before conjugation, and to determine the biodistribution of the conjugate, free antibody and free enzyme in mice.

Alternatively, the compound may be produced as a fusion compound by recombinant DNA techniques whereby a length of DNA comprises respective regions encoding the two portions of the compound of the invention either adjacent one another or separated by a region encoding a linker peptide which does not destroy the desired properties of the compound. Conceivably, the two portions of the compound may overlap wholly or partly. The DNA is then expressed in a suitable host in known ways.

The compounds of the invention are administered in any suitable way, usually parenterally, for example intravenously, intraperitoneally or, preferably (for bladder cancers), intravesically (ie into the bladder), in standard sterile, non-pyrogenic formulations of diluents and carriers, for example isotonic saline (when administered intravenously). Once the compound has bound to the target cells and been cleared from the bloodstream (if necessary), which typically takes a day or so, the pro-drug is administered, usually as a single infused dose. If needed, because the compound of the invention may be immunogenic, cyclosporin or some other immunosuppressant can be administered to provide a longer period for treatment but usually this will not be necessary.

The timing between administrations of antibody-enzyme conjugate and amygdalin may be optimised in a non-inventive way since tumour/normal tissue ratios of conjugate (at least following intravenous delivery) are highest after about 4 - 6 days, whereas at this time the absolute amount of antibody bound to the tumour, in terms of percent of injected dose per gram, is lower than at earlier times. Therefore, the optimum interval between administration of the conjugate and the pro-drug will be a compromise between peak tumour concentration of enzyme and the best distribution ratio between tumour and normal tissues.

The dosage will be chosen by the physician according to the usual criteria. At least in the case of methods employing intravenous amygdalin as the toxic pro-drug, 1 to 50 daily doses of 0.1 to 10.0 grams per square metre of body surface area, preferably 1.0-5.0 g/m² are likely to be appropriate. For oral therapy, three doses per day of 0.05 to 10.0g, preferably 1.0-5.0g, for one to fifty days may be appropriate. The dosage of the compound of the invention will similarly be chosen according to normal criteria, particularly with reference to the type, stage and location of the tumour and the weight of the patient. The duration of treatment will depend in part upon the rapidity and extent of any immune reaction to the antibody or enzyme component of the compound.

The compounds of the invention, together with an appropriate pro-drug, are in principle suitable for the destruction of cells in any tumour or other defined class of cells selectively exhibiting a recognisable (surface) entity, provided only that the cells (or neighbouring ones which one wishes to kill) are respiring aerobically, since cyanide is toxic by virtue of interfering with the electron transport pathway in oxidative phosphorylation. The compounds are principally intended for human use but could be used for treating other mammals, including dogs, cats, cattle, horses, pigs and sheep.

The methods of the invention may be particularly suitable for the treatment of bladder carcinoma *in situ*, administering the antibody-enzyme conjugate and the amygdalin intravesically. Our studies on the administration of radiolabelled antibodies via this route indicate that high tumour/normal bladder ratios can be achieved, and that the antibody does not enter the circulation. Bladder cancer accounts for 2% of all human malignancies, of which approximately 70% of cases are superficial at the time of diagnosis. Recurrences occur in as many as 80% of cases after surgical resection, 10% of these progressing to a higher grade carcinoma with poorer prognosis (Newling 1990). Any amygdalin which is able to enter the circulation will not be converted to cyanide, as there is no mammalian enzyme which catalyses this hydrolysis, and the conjugate will not enter the circulation from the bladder. If a small amount of free cyanide is able to enter the circulation it will be rapidly detoxified to thiocyanate and excreted.

The compound of the invention is administered and, once there is an optimum balance between (i) the tumour to normal cell ratio of compound and (ii) the absolute level of compound associated with the tumour, the pro-drug is administered either systemically (eg intravenously) or intravesically, into the bladder.

The invention will now be illustrated by the following examples.

### EXAMPLE 1

Materials. Almond emulsion β-D-glucosidase (BDH Chemical Co, Poole, Dorset, UK). Monoclonal antibodies H17E2 (obtainable from the Imperial Cancer Research Fund, London, UK or from Unipath, Bedford, UK) and HMFG1. H17E2 is a murine monoclonal antibody of the IgG1 subclass. It was raised in BALB/c mice immunised with human term-placental plasma membranes, and recognizes human placental alkaline phosphatase (PLAP) as well as the leucine-inhibitable form of the enzyme found at low levels in the healthy testis. H17E2 reacts strongly with neoplasms of the testis, ovary and cervix, and with the PLAP-secreting cell line used in the present studies.

HMFG1, also IgG1, was raised in BALB/c mice using delipidated human milk fat globule as immunogen (Taylor-Papadimitriou *et al*, 1981 *Int*. *J*. *Cancer* **28**, 17-21). It is directed to a determinant on the carbohydrate side chains of a high molecular weight (Mᵣ>400,000) glycoprotein normally produced by the lactating human mammary epithelial cell, but also found on some carcinomas, such as those of breast, ovary, lung and the gastrointestinal tract.

Protein conjugation. The antibody and enzyme were conjugated using the heterobifunctional cross-linker m-maleimidobenzoyl-N-hydroxysulfosuccinimide ester (MBS) (Pierce Chemical Company, Rockford, IL, USA). The β-glucosidase was dissolved in 0.05 M phosphate buffer (pH 8) to a concentration of 5 mg/ml, and then MBS, dissolved in the same buffer, was added at a molar ratio of 20:1 (MBS:enzyme) and the mixture allowed to react at room temperature for 1 hr. The unreacted MBS was removed by G-25 gel filtration (Pharmacia, Uppsala, Sweden) using 0.05 M sodium acetate containing 1 mM ethylenediaminetetraacetate (EDTA) and 0.15 M sodium chloride (pH 6.5) as elution buffer. The derivatised enzyme was concentrated in an ultrafiltration cell (Amicon, Danvers, MA, USA) to 5 mg/ml and stored under nitrogen at 4°C.

Both the specific and control antibodies were thiolated using 2-iminothiolane hydrochloride (2IT) (Sigma) in 0.1M triethanolamine buffer (pH 8.7) for 1 h at room temperature, at a molar ratio of 50:1 (2IT:antibody). The thiolated antibody was separated from the free 2IT using a G-25 column and the same elution buffer as above. The protein-containing fractions were run directly into the derivatised enzyme, concentrated to 5 mg/ml and reacted under nitrogen at 4°C overnight. An enzyme:antibody molar ratio of 2:1 was employed in order to ensure that no unconjugated antibody remained after the reaction. It was therefore possible to separate the antibody-enzyme conjugate from free β-glucosidase on a protein A-Sepharose column (Pharmacia), using phosphate buffer (pH 8) to wash the free β-glucosidase from the column, and citrate buffer (pH 6) to remove the bound antibody-enzyme conjugate.

The purity of the conjugate before and after protein A-Sepharose was tested using fast protein liquid chromatography (FPLC) on a Superose-6 column (Pharmacia). The molecular weight of the conjugate was estimated usind sodium dodecyl sulphate polyacrylamide gel electrophoresis (SDS-PAGE). The conjugate was tested for dual biological activities using an enzyme-linked immunosorbent assay (ELISA) on plates coated with antigen (PLAP), and using the enzyme assay described below.

Cytotoxicity *in vitro*. The human epidermoid carcinoma cell line H.Ep-2 was originally obtained from a metastatic nodule in the neck of a male patient with a primary tumour of the larynx. It was established in tissue culture after two generations in X-irradiated, cortisone-treated rats. Immunocytochemistry has shown the cells to bind the H17E2 antibody, but not HMFG1, which was used as a negative control in these studies. Cells were cultured in RPMI 1640 tissue culture medium supplemented with 10% foetal calf serum (Gibco Ltd, Paisley, Scotland). For cell survival experiments, after harvesting with 0.02% EDTA, 5 x 10⁵ cells were plated in quadruplicate into 25-cm² Falcon flasks (Becton Dickinson, New Jersey, USA). Twenty-four hours later the cytotoxic agent was added (either amygdalin alone, β-glucosidase alone, amygdalin plus β-glucosidase, potassium cyanide alone, amygdalin plus H17E2-β-glucosidase conjugate or amygdalin plus HMFG1-β-glucosidase conjugate). After 24 h exposure, the medium containing the drug was replaced with fresh medium and the cells allowed to grow for a further 24 h before counting by haemacytometry, using trypan blue exclusion as the criterion for cell integrity.

Flow cytometry. After incubation with the specific conjugate, H.Ep-2 cells were tested for the presence of H17E2 antibody and β-glucosidase using flow cytometry. The cells were washed twice in tissue culture medium and resuspended at a concentration of 5 x 10⁶ cells/ml. The presence of H17E2 on the surface of cells was tested by incubating 100 µl of cell suspension with F(ab')₂ fragments of a rabbit anti-mouse-IgG antibody conjugated to fluorescein isothiocyanate (FITC) (Dako, High Wycombe, Bucks) for 30 min on ice. The presence of the enzyme was tested by incubating an aliquot of the cell suspension with a polyclonal rabbit anti-β-glucosidase antibody produced in our laboratory.

After 1 h incubation, the cells were incubated with FITC-conjugated swine anti-rabbit-IgG antibody (Dako) for 30 min on ice. The FITC on the cell surface was measured using an EPICS V flow cytometer.

Enzyme assay. The β-glucosidase activity was assayed in 0.05 M acetate buffer (pH 6) using 2-nitrophenyl-β-D-glucopyranoside (BDK Chemical Co) as the chromogenic substrate. The assay was performed in 96-well plates and the reaction stopped after 10 min by the addition of 0.2 M sodium carbonate (pH 12). The absorbance of the product (2-nitrophenol) was measured at 405 mm in a Titertek Multiskan MCC/340 plate reader (Flow Laboratories, Helsinki, Finland).

### Results

The cytotoxicity *in vitro* of the H.Ep-2 cell line to amygdalin alone, amygdalin in the presence of 0.35 µM or 3.5 µM β-glucosidase, and potassium cyanide alone was measured. The IC₅₀ (dose required to give 50% cell survival) for each of these agents was found to be 114 ± 8, 0.6 ± 0.3, 0.5 ± 0.2 and 0.2 ± 0.1 mM, respectively. This indicates that although amygdalin is non-toxic to the cells except at very high concentrations, it is readily converted to the toxic compound by β-glucosidase at physiological pH and temperature, and is then able to kill cells almost as effectively as cyanide itself.

Analysis of the conjugate using FPLC showed a single peak which eluted earlier than the free antibody or enzyme. SDS-PAGE analysis of the same sample indicated that this peak corresponded to a molecular weight slightly higher than 200,000, consistent with the expected value for the β-glucosidase-antibody conjugate.

An assay of the immunoreactivity of the H17E2 antibody and the conjugate showed only slight loss of ability to bind PLAP after conjugation to β-glucosidase.

Table 1 shows the results of an enzyme assay of the H17E2-β-glucosidase conjugate at 100 nM, and the native enzyme at three concentrations. The mean Kₘ of the β-glucosidase at the three concentrations was determined to be 9.4 mM, and that of the conjugate to be 10.6 mM. Since the Vₘₐₓ for the conjugate and enzyme at the same concentration was unchanged, the increase in Kₘ indicates a loss of enzyme activity of 13%.

**Table 1**

| Enzyme assay of β-glucosidase and H17E2-β-glucosidase conjugate using 2-NPG as substrate. Values of Kₘ and Aₘₐₓ were obtained from Lineweaver-Burk plots. | | | |
|---|---|---|---|
| Sample | Concentration (nM) | Kₘ (mM) | Aₘₐₓ |
| β-glucosidase | 20 | 9.4 | 0.5 |
| β-glucosidase | 50 | 9.3 | 1.3 |
| β-glucosidase | 100 | 9.5 | 2.5 |
| H17E2-conjugate | 100 | 10.6 | 2.5 |

Flow cytometry of cells incubated with H17E2-β-glucosidase conjugate showed that both antibody and enzyme were present on the cell surface, 99% of cells being positive for both proteins. Similar results were achieved with immunocytochemistry.

In terms of the cytotoxic effect on H.Ep-2 cells of β-glucosidase alone or in the presence of 1 mM amygdalin, the enzyme alone is non-toxic up to much higher concentrations than are necessary to activate amygdalin (ie 0.35 µM). As a comparison at one enzyme concentration (50 nM) of the antibody-enzyme conjugate or an unconjugated mixture of H17E2 and β-glucosidase in the presence of 1 mM amygdalin, it was found that the treated/control cell survival ratio is 0.68 ± 0.15 for the unconjugated mixture compared with 0.39 ± 0.09 for the conjugate (mean ± standard deviation, p<0.05). In order to achieve cell killing at this level with unconjugated β-glucosidase, a 10-fold increase in enzyme concentration is necessary. The effect on H.Ep-2 cells of varying concentrations of amygdalin activated by 50 nM β-glucosidase alone, 50 nM specific H17E2-β-glucosidase conjugate or 50 nM control HMFG1-β-glucosidase conjugate was measured. The HMFG1-conjugate displayed no significant advantage over unconjugated β-glucosidase at any of the amygdalin concentrations (0.1, 1.0, 10.0 mM). The cytotoxicity of the specific conjugate at both amygdalin concentrations (1.0, 10.0 mM) is significantly greater than that of the control conjugate or the unconjugated enzyme plus amygdalin at the same concentration (p<0.05). This corresponds to approximately a 5-fold reduction in the concentration of amygdalin required to produce the same cytotoxicity.

### Summary

Amygdalin was more cytotoxic in the presence of the specific antibody-enzyme conjugate than the control conjugate or an unconjugated mixture of antibody and enzyme plus amygdalin at the same concentration, due to the cell-bound enzyme releasing a high concentration of cyanide in the tumour cell vicinity. This overcomes the problems of antigen heterogeneity and the necessity for internalisation of the cytotoxic compound, which limit the cytotoxicity of antibody-drug immunoconjugates. For example, a methotrexate-antibody complex has previously been found to be less potent than the free drug for human colon carcinoma cells *in vitro*, even with 5 molecules of methotrexate per conjugate.

Since the pro-drug is relatively non-toxic, and the enzyme is localized to the tumour site, with no endogenous homologue activity, multiple injections of prodrug may be administered while the antibody-enzyme conjugate remains bound to tumour cells, thereby increasing the differential (drug concentration x time) between tumour and normal tissues. Any free cyanide which diffuses away from the tumour site will be detoxified to thiocyanate by rhodanase, a widely distributed mammalian enzyme found in high concentrations in liver and kidney.

### EXAMPLE 2

The antibody used in this Example is not a compound of the invention. Patients and methods. 23 patients undergoing cystoscopy for known or suspected bladder carcinoma entered the study. Each patient gave written consent before entering the study. Four patients were studied twice and one three times: a total of 29 instillations were performed.

150-200 µg of AUA1 or 11.4.1. monoclonal antibody, labelled with 0.3-0.5 mCi of ¹¹¹In and diluted in 50 ml of 0.9 g per 100 ml NaCl solution were administered intravesically through a catheter. AUA1 was administered in 22 and 11.4.1. in 7 cases. The radiolabelled antibody was kept in the bladder for 1 h during which patients were encouraged to change position every 15 min. The bladder was then emptied and washed twice with 50 ml of 0.9 g per 100 ml NaCl solution. AUA1 monoclonal antibody is an IgG1 mouse immunoglobulin recognising a 35 kDa glycoprotein present on the membrane of various types of epithelial cells. It reacts with a restricted number of normal epithelial tissues and most human carcinomas where it is expressed more intensely than in normal tissues (Spurr, N. K. *et al* (1986) *Br*. *J*. *Cancer*. **38**, 631-636). 11.4.1. monoclonal antibody is an IgG1 mouse immunoglobulin which recognises an HLA component of mouse lymphocytes and does not react with any human tissues. It was used as a negative control (Oi, V. T. *et al* (1979) *Curr*. *Topic*. *Microbiolo*. *Immunol*. **81**, 115-110).

The monoclonal antibodies were coupled with DTPA (Sigma, UK) using the method of Hnatowich *et al* ((1983) *J*. *Immunol*. *Meth*. 65, 147-152). Briefly, DTPA was added to the antibodies (10 mg/ml) at a molar ratio 10:1. The reaction was carried out at room temperature and pH 8.0 for 10 min. Free DTPA was removed using a Sephadex G50 column. Protein containing fractions were collected, Millipore filtered and stored at -70°C. ¹¹¹In (Amersham, UK) was added to DTPA coupled antibodies and reacted for 30 min at room temperature at pH 6.5. Free ¹¹¹In was removed using a Sephadex G50 column. 60%-70% of ¹¹¹In was bound to AUA1 and 85%-90% to control antibody. The specific activity ranged from 2-3 mCi/mg. Immunoreactivity of AUA1 before and after administration into the bladder was tested using an ELISA and competition RIA. *In vivo* stability of both antibodies was tested by SDS-PAGE and gel autoradiography. Cystoscopy was performed at 2 h (n=14), 24 h (n=12) and 48 h (n=3) after instillation. Samples from tumours as well as from normal areas were taken during cystoscopy. They were fresh frozen or fixed in Methacarn. Samples were weighed and then counted in a gamma-counter for radioactivity. They were then processed and embedded in paraffin. Blood samples were obtained at 2h, 1, 2, and 3 days after the instillation and counted for radioactivity. Haematoxylin-Eosin and a two step immunoperoxidase method using AUA1 and control antibody were performed on paraffin and frozen sections. Data were analysed statistically using the Student's t-test.

ELISA and RIA showed that instillation into the urinary bladder caused no loss of AUA1 immunoreactivity. Autoradiography of the gel after instillation showed that practically all ¹¹¹In was bound to the antibodies.

Tumour was found in 20 out of 29 cases studied (Tis:1, Ta:17, T1:1, T2:1; Grade I:9, Grade II:6, Grade III:4). Eighteen of the tumours were papillary, one anaplastic and one was *in situ* carcinoma. The samples taken from normal areas consisted of normal urothelium with chronic inflammatory infiltration in a few of these cases. In one case (instillation performed 2h before the cystoscopy) no normal samples were taken. Immunohistochemical analysis showed a weak reaction of AUA1 with the basal layer of the normal urothelium while all the tumours showed a more intense reaction. The intensity of the staining and the number of the positive cells increased with the grading of the tumours. Grade I tumours reacted only on the lower third of the epithelium at a depth of 1-2 cells while Grade II tumours reacted more strongly on the lower and the middle third of the epithelium. Grade III tumours showed intense reaction of 70%-100% of the cells. *In situ* carcinoma was also positive. Immunostaining using 11.4.1. was negative in all cases.

Radioactivity targeted on the tumours and normal tissues, at various time points after AUA1 administration, showed that the uptake by the tumours, in all cases, was higher than that of normal tissues of the same patients. The mean uptake of both antibodies on tumour and non-tumour samples is shown in Table 1.

**Table 1**

| **Mean uptake of tumour**^{**a**} **and non-tumour tissues.** 150-200 µg of AUA1 or 11.4.1. labelled with 0.3-0.5 mCi of ¹¹¹In were administered intravesically and remained in the bladder for 1h. Cystoscopy was performed at 2h, 24h and 48h after the instillation. Tumour and non-tumour samples were taken and counted for radioactivity. The presence of tumour in the samples was determined after histological examination. | | | |
|---|---|---|---|
| Time after instillation (h) | 2 | 24 | 48 |
| Antibody | | AUA1 | |
| Tumour | 6.12±5.50 (n=6) | 1.70±2.57 (n=7) | 0.30±0.17 (n=3) |
| Non-tumour | 0.32±0.50 (n=10) | 0.22±0.30 (n=8) | 0 (n=3) |
| Antibody | | 11.4.1. | |
| Tumour | 0.075±0.075 (n=2) | 0.025±0.025 (n=2) | - |
| Non-tumour | 0.30±0.42 (n=3) | 0.15±0.26 (n=4) | - |

| | | | |
|---|---|---|---|
| ^{**a**}**expressed as 10**^{**3**} **x percentage of injected dose/g of tissue** | | | |

It must be emphasised that in 5 out of 9 cases at 2 h, 4 out of 8 at 24 h and in all 3 cases at 48 h there was no uptake in the non-tumour samples. When 11.4.1. was administered no activity was found in tumour samples in 2 out of 4 cases while in the remaining 2 cases the uptake was more than 30-fold lower than the respective values for specific antibody. There was no radioactivity in the blood of the patients at any of the time points they were counted. Statistical analysis of the results show that there is significant difference between the uptake of AUA1 in tumour and non-tumour samples at 2 h and 48 h (p<0.05) after the instillation and between the tumour uptake of AUA1 and 11.4.1. at 2 h (p<0.05). The correlation between tumour uptake of AUA1 and the grade of the tumours is shown in Table 2. The uptake increased with the grade of the tumours. There is a significant difference between the uptake by the normal tissue and the Grade II tumours at both 2 h (p<0.05) and 24 h (p<0.001) after the instillation.

**Table 2**

| **Correlation of the grade of the tumours with the mean uptake**^{**a**} **of AUA1.** | | | |
|---|---|---|---|
| Time after instillation (h) | 2 | 24 | 48 |
| Grade | | | |
| I | 0.2 (n=1) | 0.36±0.49 (n=4) | 0.40±0.13 (n=2) |
| II | 4.9±2.8 (n=4) | 1.55±0.25 (n=2) | - |
| III | 17 (n=1) | 7.35 (n=1) | - |
| *In situ* | | | 0.10 (n=1) |

| | | | |
|---|---|---|---|
| ^{**a**}**expressed as 10**^{**3**} **x percentage of injected dose/g of tissue.** | | | |

This difference in tumour and normal uptake is favourable for immunotherapy and in fact represents one of the best achieved by using monoclonal antibodies up to date. The correlation of the uptake of the specific antibody with the grade of the tumours represents an advantage of this approach since the frequency of recurrence and invasion increases with the grade of the tumours. Grade II and especially Grade III tumours need additional treatment apart from the surgical excision. Grade I tumour uptake showed no difference from the uptake of the normal tissues. This is probably due to the restriction of the antigenic expression of AUA1 in the basal layer of the epithelium which is difficult for the antibody to reach. Other antibodies recognising epitopes on more accessible tumour cells would probably be more suitable for these tumours.

Human anti-mouse antibody responses and myelosuppression due to the accumulation of immunoconjugates by the reticuloendothelial system represent the most serious drawbacks when monoclonal antibodies are used for cancer therapy. Intravesical administration of radiolabelled AUA1 antibody shows that there is no circulating antibody in the blood because there is no or very limited absorption from the normal urothelium. Large molecules are most unlikely to be absorbed even when employed in high doses in contrast to chemotherapeutic drugs which can diffuse into the systemic circulation and produce myelosuppression. Using our approach systemic toxicity may be avoided and repeated administrations may be given. The advantages of selective uptake of the specific antibody and its non-toxic potential also make this method ideal for the two step approach (targeted conversion of non-toxic prodrug to toxic drug) described above. These exploit the selective uptake of the antibody to deliver an enzyme or streptavidin (as an antibody-enzyme or antibody-biotin immunoconjugate) to tumour cells. After the clearance of the conjugate from the circulation and normal organs an inactive prodrug or radioactive streptavidin is administered. The cleavage of the prodrug by the enzyme results in the generation of a toxic agent. When the streptavidin-biotin system is used, the high affinity of these substances for each other is exploited in order to deliver the radioisotope to the tumour. In the method of this Example, the interval between the two steps can be very short because there is no circulating antibody and very low or no uptake by the normal urothelium.

## Claims (Claims for the following Contracting State(s): AT, BE, CH, DE, DK, FR, GB, IT, LI, LU, NL, SE)

1. A compound comprising a target cell specific portion and an enzymatically active portion, the enzymatically active portion being capable of generating cyanide from a cyanogenic pro-drug provided that the said enzymatically active portion is not a carbohydrate-cleaving enzyme capable of converting a glycosylated pro-drug into a free drug.

2. A compound according to Claim 1 wherein the enzymatically active portion comprises at least the catalytic portion of a hydroxynitrile lyase.

3. A compound according to any one of the preceding claims wherein the target cell specific portion comprises an antibody or part thereof.

4. A compound according to any one of the preceding claims wherein the cell specific portion recognises and selectively binds to a tumour cell antigen.

5. A bispecific antibody capable of binding to a target cell specific antigen and to an enzymatically active molecule, characterised in that the said molecule is capable of generating cyanide from a cyanogenic pro-drug provided that said enzymatically active portion is not a carbohydrate-cleaving enzyme capable of converting a glycosylated pro-drug into a free drug.

6. A therapeutic system comprising:
(i) a compound comprising a target cell specific portion and an enzymatically active portion, characterised in that the enzymatically active portion is capable of generating cyanide from a cyanogenic pro-drug, and
(ii) a cyanogenic pro-drug which can be cleaved by the said enzymatic portion to yield cyanide.

7. A therapeutic system according to Claim 6 wherein the cyanogenic pro-drug is a mono- or di-saccharide.

8. A therapeutic system according to Claim 7 wherein the cyanogenic pro-drug is amygdalin.

9. A pharmaceutical composition for parenteral delivery comprising a compound according to any one of Claims 1 to 4.

10. A compound according to any one of Claims 1 to 5 for use in medicine.

11. Use of a compound comprising a target cell specific portion and an enzymatically active portion, characterised in that the enzymatically active portion is capable of generating cyanide from a cyanogenic pro-drug in the manufacture of a medicament for treating a mammal having target cells to be destroyed, wherein the treatment comprises (1) administering said compound to the mammal, (2) allowing the ratio of (compound bound to the target cells):(compound not bound to the target cells) to reach a desired value, and (3) administering a cyanogenic pro-drug which can be cleaved by the said enzymatically active portion to yield cyanide.

12. Use according to Claim 11 wherein the said compound and the cyanogenic pro-drug are administered directly into the bladder of the mammal.

## Claims (Claims for the following Contracting State(s): ES, GR)

1. A method of making a compound comprising a target cell specific portion and an enzymatically active portion, the enzymatically active portion being capable of generating cyanide from a cyanogenic pro-drug provided that the said enzymatically active portion is not a carbohydrate-cleaving enzyme capable of converting a glycosylated pro-drug into a free drug comprising the step of combining the said target cell specific portion and the said enzymatically active portion.

2. A method according to Claim 1 wherein the enzymatically active portion comprises at least the catalytic portion of a hydroxynitrile lyase.

3. A method according to any one of the preceding claims wherein the target cell specific portion comprises an antibody or part thereof.

4. A method according to any one of the preceding claims wherein the cell specific portion recognises and selectively binds to a tumour cell antigen.

5. A method of making a bispecific antibody capable of binding to a target cell specific antigen and to an enzymatically active molecule, characterised in that the said molecule is capable of generating cyanide from a cyanogenic pro-drug provided that said enzymatically active portion is not a carbohydrate-cleaving enzyme capable of converting a glycosylated pro-drug into a free drug comprising the step of combining a binding site for the said target cell specific antigen and a binding site for the said enzymatically active molecule.

6. A therapeutic system comprising:
(i) a compound comprising a target cell specific portion and an enzymatically active portion, characterised in that the enzymatically active portion is capable of generating cyanide from a cyanogenic pro-drug, and
(ii) a cyanogenic pro-drug which can be cleaved by the said enzymatic portion to yield cyanide.

7. A therapeutic system according to Claim 6 wherein the cyanogenic pro-drug is a mono- or di-saccharide.

8. A therapeutic system according to Claim 7 wherein the cyanogenic pro-drug is amygdalin.

9. A method of making a pharmaceutical composition for parenteral delivery comprising combining a compound obtainable by the method of any one of Claims 1 to 4 and a pharmaceutically acceptable carrier.

10. A compound obtainable by the method of any one of Claims 1 to 5 for use in medicine.

11. Use of a compound comprising a target cell specific portion and an enzymatically active portion, characterised in that the enzymatically active portion is capable of generating cyanide from a cyanogenic pro-drug in the manufacture of a medicament for treating a mammal having target cells to be destroyed, wherein the treatment comprises (1) administering said compound to the mammal, (2) allowing the ratio of (compound bound to the target cells):(compound not bound to the target cells) to reach a desired value, and (3) administering a cyanogenic pro-drug which can be cleaved by the said enzymatically active portion to yield cyanide.

12. Use according to Claim 11 wherein the said compound and the cyanogenic pro-drug are administered directly into the bladder of the mammal.

## Patentansprüche (Patentansprüche für folgende(n) Vertragsstaat(en): AT, BE, CH, DE, DK, FR, GB, IT, LI, LU, NL, SE)

1. Verbindung mit einem für eine Zielzelle spezifischen Bereich und einem enzymatisch aktiven Bereich, wobei der enzymatisch aktive Bereich die Fähigkeit zur Bildung eines Cyanids aus einem cyanogenen Promedikament besitzt und es sich bei dem enzymatisch aktiven Bereich nicht um ein kohlenhydratspaltendes Enzym mit der Fähigkeit zur Umwandlung eines glykosylierten Promedikaments in ein freies Medikament handelt.

2. Verbindung nach Anspruch 1, wobei der enzymatisch aktive Bereich mindestens den katalytischen Bereich einer Hydroxynitrillyase umfaßt.

3. Verbindung nach einem der vorhergehenden Ansprüche, wobei der für eine Zielzelle spezifische Bereich einen Antikörper oder einen Teil desselben umfaßt.

4. Verbindung nach einem der vorhergehenden Ansprüche, wobei der für die Zelle spezifische Bereich ein Tumorzellenantigen erkennt und eine selektive Bindung damit eingeht.

5. Bispezifischer Antikörper mit der Fähigkeit zur Bindung an ein für eine Zielzelle spezifisches Antigen und an ein enzymatisch aktives Molekül, dadurch gekennzeichnet, daß das Molekül die Fähigkeit zur Erzeugung eines Cyanids aus einem cyanogenen Promedikament besitzt, wobei gilt, daß es sich bei dem enzymatisch aktiven Bereich nicht um ein kohlenhydratspaltendes Enzym mit der Fähigkeit zur Umwandlung eines glykosylierten Promedikaments in ein freies Medikament handelt.

6. Therapeutisches System, umfassend:
(I) eine Verbindung mit einem für eine Zielzelle spezifischen Bereich und einem durch die Fähigkeit zur Erzeugung eines Cyanids aus einem cyanogenen Promedikament gekennzeichneten, enzymatisch aktiven Bereich, und
(II) ein cyanogenes Promedikament, welches durch den enzymatischen Teil unter Cyanidbildung gespalten werden kann.

7. Therapeutisches System nach Anspruch 6, wobei es sich bei dem cyanogenen Promedikament um ein Mono- oder Disaccharid handelt.

8. Therapeutisches System nach Anspruch 7, wobei es sich bei dem cyanogenen Promedikament um Amygdalin handelt.

9. Arzneimittelzubereitung zur parenteralen Abgabe, umfassend eine Verbindung nach einem der Ansprüche 1 bis 4.

10. Verbindung nach einem der Ansprüche 1 bis 5 zur Verwendung in der Medizin.

11. Verwendung einer Verbindung mit einem für eine Zielzelle spezifischen Bereich und einem durch die Fähigkeit zur Erzeugung eines Cyanids aus einem cyanogenen Promedikament gekennzeichneten, enzymatisch aktiven Bereich bei der Herstellung eines Medikaments zur Behandlung eines Säugetiers, das zu zerstörende Zielzellen aufweist, wobei man bei der Behandlung
(1) die betreffende Verbindung an das Säugetier verabreicht;
(2) das Verhältnis (an die Zielzellen gebundene Verbindung)/(an die Zielzellen nicht gebundene Verbindung) einen gewünschten Wert erreichen läßt und
(3) ein durch den enzymatisch aktiven Bereich unter Cyanidbildung spaltbares cyanogenes Promedikament verabreicht.

12. Verwendung nach Anspruch 11, wobei die Verbindung und das cyanogene Promedikament direkt in die (Harn)Blase des Säugetiers verabreicht werden.

## Patentansprüche (Patentansprüche für folgende(n) Vertragsstaat(en): ES, GR)

1. Verfahren zur Herstellung einer Verbindung mit einem für eine Zielzelle spezifischen Bereich und einem enzymatisch aktiven Bereich, wobei der enzymatisch aktive Bereich die Fähigkeit zur Erzeugung eines Cyanids aus einem cyanogenen Promedikament besitzt und es sich bei dem enzymatisch aktiven Bereich nicht um ein kohlenhydratspaltendes Enzym mit der Fähigkeit zur Umwandlung eines glykosylierten Promedikaments in ein freies Medikament handelt, durch Vereinigen des für die Zielzelle spezifischen Bereichs mit dem enzymatisch aktiven Bereich.

2. Verfahren nach Anspruch 1, wobei der enzymatisch aktive Bereich mindestens den katalytischen Bereich einer Hydroxynitrillyase umfaßt.

3. Verfahren nach einem der vorhergehenden Ansprüche, wobei der für die Zielzelle spezifische Bereich einen Antikörper oder einen Teil desselben umfaßt.

4. Verfahren nach einem der vorhergehenden Ansprüche, wobei der für die Zelle spezifische Bereich ein Tumorzellenantigen erkennt und eine selektive Bindung damit eingeht.

5. Verfahren zur Herstellung eines bispezifischen Antikörpers mit der Fähigkeit zur Bindung an ein für eine Zielzelle spezifisches Antigen und an ein enzymatisch aktives Molekül, dadurch gekennzeichnet, daß das Molekül die Fähigkeit zur Erzeugung eines Cyanids aus einem cyanogenen Promedikament besitzt, wobei gilt, daß es sich bei dem enzymatisch aktiven Bereich nicht um ein kohlenhydratspaltendes Enzym mit der Fähigkeit zur Umwandlung eines glykosylierten Promedikaments in ein freies Medikament handelt, durch Vereinigen einer Bindungsstelle für das für die Zielzelle spezifische Antigen mit einer Bindungsstelle für das enzymatisch aktive Molekül.

6. Therapeutisches System, umfassend:
(I) eine Verbindung mit einem für eine Zielzelle spezifischen Bereich und einem durch die Fähigkeit zur Erzeugung eines Cyanids aus einem cyanogenen Promedikament gekennzeichneten, enzymatisch aktiven Bereich, und
(II) ein cyanogenes Promedikament, welches durch den enzymatischen Bereich unter Cyanidbildung gespalten werden kann.

7. Therapeutisches System nach Anspruch 6, wobei es sich bei dem cyanogenen Promedikament um ein Mono- oder Disaccharid handelt.

8. Therapeutisches System nach Anspruch 7, wobei es sich bei dem cyanogenen Promedikament um Amygdalin handelt.

9. Verfahren zur Herstellung einer Arzneimittelzubereitung zur parenteralen Abgabe durch Vereinigen einer nach dem Verfahren gemäß einem der Ansprüche 1 bis 4 erhältlichen Verbindung mit einem pharmazeutisch akzeptablen Träger.

10. Verbindung, erhältlich nach dem Verfahren gemäß einem der Ansprüche 1 bis 5 zur Verwendung in der Medizin.

11. Verwendung einer Verbindung mit einem für eine Zielzelle spezifischen Bereich und einem durch die Fähigkeit zur Erzeugung eines Cyanids aus einem cyanogenen Promedikament gekennzeichneten, enzymatisch aktiven Bereich bei der Herstellung eines Medikaments zur Behandlung eines Säugetiers, das zu zerstörende Zielzellen aufweist, wobei man bei der Behandlung
(1) die betreffende Verbindung an das Säugetier verabreicht;
(2) das Verhältnis (an die Zielzellen gebundene Verbindung)/(an die Zielzellen nicht gebundene Verbindung) einen gewünschten Wert erreichen läßt und
(3) ein durch den enzymatisch aktiven Bereich unter Cyanidbildung spaltbares cyanogenes Promedikament verabreicht.

12. Verwendung nach Anspruch 11, wobei die Verbindung und das cyanogene Promedikament direkt in die (Harn)Blase des Säugetiers verabreicht werden.

## Revendications (Revendications pour l'(les) Etat(s) contractant(s) suivant(s): AT, BE, CH, DE, DK, FR, GB, IT, LI, LU, NL, SE)

1. Composé comprenant une partie spécifique à une cellule cible et une partie enzymatique active, la partie enzymatique active étant capable de générer du cyanure à partir d'une prodrogue cyanogène à la condition que ladite partie active du point de vue enzymatique ne soit pas une enzyme dissociant les hydrates de carbone capable de convertir une prodrogue glycosylée en une drogue libre.

2. Composé selon la revendication 1, dans lequel la partie enzymatique active comprend au moins la partie catalytique d'une lyase hydroxynitrile.

3. Composé selon l'une quelconque des revendications précédentes, dans lequel la partie spécifique à une cellule cible comprend un anticorps ou une partie d'anticorps.

4. Composé selon l'une quelconque des revendications précédentes, dans lequel la partie spécifique à une cellule reconnait, et se lie sélectivement à, un antigène de cellule tumorale.

5. Anticorps bispécifique capable de se lier à un antigène spécifique à une cellule cible et à une molécule enzymatique active, caractérisé en ce que ladite molécule est capable de générer du cyanure à partir d'une prodrogue cyanogène à la condition que ladite partie enzymatique active ne soit pas une enzyme dissociant les hydrates de carbone capable de convertir une prodrogue glycosylée en une drogue libre.

6. Système thérapeutique comprenant:
(i) un composé comprenant une partie spécifique à une cellule cible et une partie enzymatique active, caractérisé en ce que la partie enzymatique active est capable de générer du cyanure à partir d'une prodrogue cyanogène, et
(ii) une prodrogue cyanogène pouvant être dissociée par ladite partie enzymatique afin de donner du cyanure.

7. Système thérapeutique selon la revendication 6, dans lequel la prodrogue cyanogène est un mono ou un disaccharide.

8. Système thérapeutique selon la revendication 7, dans lequel la prodrogue cyanogène est de l'amygdaline.

9. Composition pharmaceutique administrable par voie parentérale comprenant un composé selon l'une quelconque des revendications 1 à 4.

10. Composé selon l'une quelconque des revendications 1 à 5 destiné à être utilisé en médecine.

11. Utilisation d'un conposé comprenant une partie spécifique à une cellule cible et une partie enzymatique activee, caractérisée en ce que la partie enzymatique active est capable de générer du cyanure à partir d'une prodrogue cyanogène, pour la fabrication d'un médicament destiné à traiter un mammifère ayant des cellules cibles à détruire, dans laquelle le traitement comprend les étapes consistant à (1) administrer ledit composé au mammifère, (2) permettre au rapport (composé lié aux cellules cibles) sur (composé non lié aux cellules cibles) d'atteindre une valeur souhaitée, et (3) administrer une prodrogue cyanogène pouvant être dissociée par ladite partie enzymatique active pour donner du cyanure.

12. Utilisation selon la revendication 11, dans laquelle ledit composé et la prodrogue cyanogène sont administrés directement dans la vessie du mammifère.

## Revendications (Revendications pour l'(les) Etat(s) contractant(s) suivant(s): ES, GR)

1. Méthode de réalisation d'un composé comprenant une partie spécifique à une cellule cible et une partie enzymatique active, la partie enzymatique active étant capable de générer du cyanure à partir d'une prodrogue cyanogène à la condition que ladite partie enzymatique active ne soit pas une enzyme dissociant les hydrates de carbone capable de convertir une prodrogue glycosylée en une drogue libre, comprenant l'étape consistant à combiner ladite partie spécifique à une cellule cible et ladite partie enzymatique active.

2. Méthode selon la revendication 1, dans laquelle la partie enzymatique active comprend au moins la partie catalytique d'une lyase hydroxynitrile.

3. Méthode selon l'une quelconque des revendications précédentes, dans laquelle la partie spécifique à une cellule cible comprend un anticorps ou une partie d'anticorps.

4. Méthode selon l'une quelconque des revendications précédentes, dans laquelle la partie spécifique à une cellule reconnaît, et se lie sélectivement à, un antigène de cellule tumorale.

5. Méthode de réalisation d'un anticorps bispécifique capable de se lier à un antigène spécifique à une cellule cible et à une molécule enzymatique active, caractérisée en ce que ladite molécule est capable de générer du cyanure à partir d'une prodrogue cyanogène à la condition que ladite partie active du point de vue enzymatique ne soit pas une enzyme dissociant les hydrates de carbone capable de convertir une prodrogue glycosylée en une drogue libre, comprenant l'étape consistant à combiner un site de liaison pour ledit antigène spécifique d'une cellule cible et un site de liaison pour ladite molécule enzymatique active.

6. Système thérapeutique comprenant:
(i) un composé comprenant une partie spécifique à une cellule cible et une partie enzymatique active, caractérisé en ce que la partie enzymatique active est capable de générer du cyanure à partir d'une prodrogue cyanogène, et
(ii) une prodrogue cyanogène pouvant être dissociée par ladite partie enzymatique afin de donner du cyanure.

7. Système thérapeutique selon la revendication 6, dans lequel la prodrogue cyanogène est un mono ou un disaccharide.

8. Système thérapeutique selon la revendication 7, dans lequel la prodrogue cyanogène est de l'amygdaline.

9. Méthode de réalisation d'une composition pharmaceutique administrable par voie parentérale comprenant la combinaison d'un composé pouvant être obtenu par la méthode de l'une quelconque des revendications 1 à 4 et un support acceptable du point de vue pharmaceutique.

10. Composé pouvant être obtenu par la méthode de l'une quelconque des revendications 1 à 5 destiné à être utilisé en médecine.

11. Utilisation d'un composé comprenant une partie spécifique à une cellule cible et une partie enzymatique active, caractérisée en ce que la partie enzymatique active est capable de générer du cyanure à partir d'une prodrogue cyanogène, pour la fabrication d'un médicament destiné à traiter un mammifère ayant des cellules cibles à détruire, dans laquelle le traitement comprend les étapes consistant à (1) administrer ledit composé au mammifère, (2) permettre au rapport (composé lié aux cellules cibles) sur (composé non lié aux cellules cibles) d'atteindre une valeur souhaitée, et (3) administrer une prodrogue cyanogène pouvant être dissociée par ladite partie enzymatique active pour donner du cyanure.

12. Utilisation selon la revendication 11, dans laquelle ledit composé et la prodrogue cyanogène sont administrés directement dans la vessie du mammifère.
